# FASCICULE DE BREVET EUROPEEN

(11) **EP 1 173 601 B1**
(45) Date de publication et mention de la délivrance du brevet: **12.11.2003**
(21) Numéro de dépôt: 00917172.9
(22) Date de dépôt: 10.04.2000
(51) Int. Cl.: C12P 19/04, A23L 1/054, C08B 37/00, C12N 1/20

(54) **HETEROPOLYSACCHARIDE PRODUIT PAR UN PSEUDOMONAS SP**
DURCH PSEUDOMONAS SP HERGESTELLTE HETEROPOLYSACCHARIDE
HETEROPOLYSACCHARIDE PRODUCED BY PSEUDOMONAS SP

(30) Priorité: 15.04.1999 FR 9904743
(43) Date de publication de la demande: 23.01.2002
(73) Titulaire: Rhodia Chimie, 92512 Boulogne-Billancourt (FR)
(72) Inventeur: VASLIN, Sophie, F-92210 Saint-Cloud (FR); SENECHAL, Alain, F-94220 Charenton (FR); CHEVALLEREAU, Paule, F-79500 Melle (FR); SIMON, Jean-Luc, F-79500 Melle (FR); CANTIANI, Robert, F-60996 Lyon (FR)
(74) Mandataire: Delenne, Marc
(86) Numéro de dépôt international: FR0000907
(87) Numéro de publication internationale: WO00063412

(56) Documents cités:
- EP-A- 0 231 585
- EP-A- 0 410 604
- EP-A- 0 534 855
- FETT F W ET AL: "Identification of exopolysaccharides produced by fluorescent pseudomonads associated with commercial mushroom (Agaricus bisporus) production." APPLIED AND ENVIRONMENTAL MICROBIOLOGY, vol. 61, no. 2, février 1995 (1995-02), pages 513-517, XP002125942 WASHINGTON,DC, US ISSN: 0099-2240
- DATABASE FSTA [en ligne] INTERNATIONAL FOOD INFORMATION SERVICE (IFIS), FRANFURT/MAIN, DE; STABNIKOVA E V ET AL: "Possible utilization of exopolysaccharide producers in the baking industry." Database accession no. 84-3-10-m1181 XP002125943 & IZVESTIYA VYSSHIKH UCHEBNYKH ZAVEDENII, PISHCHEVAYA TEKHNOLOGIYA, 1983, Kievskii Ordena Trudovogo Krasnogo Znameni Tekh. Inst. Pishchevoi Promyshlennosti, Kiev, USSR

## Description

La présente invention concerne un nouveau hétéropolysaccharide (HP), son procédé de préparation par fermentation d'une souche *Pseudomonas sp I - 2054* (ou *DSM 12295*), ladite souche, et les utilisations de cet hétéropolysaccharide en tant qu'agent épaississant et/ou gélifiant.

Dans de nombreux domaines industriels, on est constamment à la recherche de nouveaux composés présentant :
- des propriétés rhéologiques améliorées et capables de former des gels,
- une compatibilité accrue avec les milieux dans lesquels ils sont incorporés,
- une grande stabilité dans une large gamme de températures et de pH.

Dans le cas des composés obtenus à l'issue d'une fermentation bactérienne, il est également important que le composé ait une bonne productivité.

La faculté de gélifier est très intéressante car ce sont des systèmes particulièrement attrayants de par la diversité des domaines dans lesquels ils trouvent des applications : certaines applications nécessitent l'utilisation d'un gel.

Ainsi, par exemple, l'industrie agroalimentaire propose une large gamme de produits gélifiés (crèmes, yoghourts, gelées diverses, glaces...), l'industrie pharmaceutique utilise les gels comme supports de principes actifs ou agents épaississants.

Dans un tout autre domaine, certaines peintures ne gouttent pas, car elles possèdent au repos des caractéristiques de gel alors qu'elles s'étalent facilement sous l'action du pinceau (profil rhéofluidifiant).

Les gels aqueux sont aussi utilisés comme supports chromatographiques ou encore pour l'élaboration de lentilles de contact.

Les hétéropolysaccharides d'origine bactérienne comme par exemple la gomme xanthane, ont déjà été décrits et utilisés pour leurs propriétés rhéologiques performantes dans des conditions de température et de pH extrêmes.

On peut également citer les hétéropolysaccharides décrit dans les demandes EP 410 604 et EP 534 855, extraits de la fermentation de deux souches différentes de pseudomonas sp et qui sont décrits comme étant des agents de viscosité et des agents épaississant.

Toutefois, ces hétéropolysaccharides qui conviennent dans des applications en solution, ne conduisent pas toujours à des gels.

Il est connu que la gélification d'un milieu a lieu lorsqu'un réseau tridimensionnel est formé suite à la réticulation des composants dudit milieu.

Habituellement, cette gélification est amenée par ajout dans le milieu de cations supplémentaires notamment de type alcalin où alcalino-terreux (par exemple le calcium et/ou le magnésium), par basculement de pH vers les pH acides ou basiques, par adjonction d'un autre composé notamment un autre polysaccharide (par exemple l'association de xanthane et de caroube), ou par modification de la température.

Quelle que soit l'application envisagée, les conditions de gélification précitées peuvent :
- nuire à la stabilité et à la compatibilité du gel final du fait des interactions entre les cations supplémentaires ou le coadditif que l'on doit introduire pour obtenir le gel et les autres ingrédients présents dans lesdites compositions, ou
- dénaturer l'hétéropolysaccharide et/ou les autres ingrédients présents dans lesdites compositions du fait des températures élevées et/ou des changements de pH.

Par "gel", dans le cadre de la présente invention, on désigne un pseudo-solide (comportement proche du solide), résultant de l'association, au moins partielle, de chaînes d'hétéropolysaccharide dispersées dans un liquide. Dans un domaine de fréquences de sollicitation ω, les gels pseudo-solides sont en général caractérisés en ce qui conceme leur composante solide par un module élastique G'(ω) appelé également module de conservation, et en ce qui conceme leur composante liquide ou visqueuse par un module visqueux G"(ω) appelé également module de perte.

Les grandeurs mécaniques G'(ω) et G"(ω) peuvent être mesurées à l'aide d'un rhéomètre à déformation imposée et fonctionnant en mode oscillatoire. A titre indicatif et non limitatif, on peut citer par exemple un rhéomètre Rheo-Fluid Spectrometer®.

G' et G" peuvent aussi être mesurées sur un rhéomètre à contrainte imposée et fonctionnant en mode oscillatoire. A titre indicatif, on peut citer par exemple un rhéomètre CARRIMED®.

Le principe de la mesure consiste à déterminer dans un premier temps le domaine de déformation mécanique réversible dans lequel la réponse du gel à la sollicitation mécanique est linéaire en fonction de ladite déformation. Dans un second temps, le gel est soumis à une valeur fixe de déformation mécanique comprise dans le domaine linéaire précédemment déterminé. C'est alors que le rhéomètre procède à un balayage en fréquence ω.

La réponse en contrainte du gel qui est en phase avec la déformation donne accès au module élastique G'(ω). G'(ω) correspond à l'énergie emmagasinée par le gel sous forme élastique et est récupérable.

La réponse en contrainte du gel qui est en déphasage d'un angle 90° avec la déformation donne accès au module visqueux G"(ω), G"(ω) correspond à l'énergie dissipée par l'écoulement visqueux et est irrécupérable.

Un gel est dit fort ou vrai lorsque dans tout le domaine de fréquence de sollicitation (ω) balayé le rapport G'/G" est supérieur ou égal à 10, c'est-à-dire lorsque l'élasticité du gel demeure forte et lorsque la valeur de G'(ω) est supérieure ou égale à 10 Pa.

La présente invention a précisément pour but de proposer des hétéropolysaccharides qui possèdent de très bonnes propriétés rhéologiques, notamment en terme de propriétés épaississantes et. pseudo-plastiques (rhéofluidifiantes) ainsi que la faculté de conduire à des gels vrais sans ajout de cations supplémentaires au milieu, sans basculement de pH, et ce à des températures inférieures ou égales à 40°C.

La présente invention a également pour but de proposer un hétéropolysaccharide présentant de très bonnes propriétés rhéoiogiques aux faibles concentrations.

La présente invention concerne d'abord un hétéropolysaccharide (HP) caractérisé en ce qu'il est susceptible d'être obtenu par fermentation d'un milieu comportant au moins une souche *Pseudomonas sp I - 2054* (ou *DSM 12295*), un de ses recombinants, ou un de ses mutants, et une source de carbone assimilable par ladite souche, l'un de ses recombinants, ou l'un de ses mutants.

La souche *Pseudomonas sp* a été déposée conformément au Traité de Budapest, auprès de la Collection Nationale de Culture des Micro-organismes (CNCM), le 22 juillet 1998, où elle est publiquement accessible sous le numéro I - 2054. Elle a également été déposée auprès de la Deutsche Sammlung von Mikroorganismen und Zellkulturen GmbH (DSMZ), le 13 juillet 1998, où elle est publiquement accessible sous le numéro DSM 12295. Cette souche constitue un des objets de l'invention.

La culture pure de *Pseudomonas sp I - 2054* (ou *DSM 12295*), qui constitue un autre aspect de la présente invention, peut être effectuée en boîte de Pétri incubée à une température comprise entre 25°C et 30°C, et plus particulièrement comprise entre 25°C et 28°C, pendant environ 24 heures.

Les sources de carbone et d'azote assimilables par le *Pseudomonas sp I - 2054* (ou *DSM 12295*), peuvent être choisies parmi le glucose, le fructose, le galactose, le tréhalose, le mannose, le mélobiose, le saccharose, le raffinose, le maltotriose, le maltose, le lactose, le lactulose, le méthyl-β-galactopyranoside, le méthyl-α-galactopyranoside, le cellobiose, le gentobiose, le méthyl-β-D-glucopyranoside, le méthyl-α-D-glucopyranoside, l'esculine, le ribose, l'arabinose, le xylose, le palatinose, le rhamnose, le fucose, le mélézitose, le D(+) arabitol, le L(-) arabitol, le xylitol, le dulcitol. le tagatose, le glycérol, le myo-innositol, le mannitol, le maltitol, le turanose, le sorbitol, l'adonitol, le lyxose, l'érythritol, le D(-) tartrate, le D(+) malate. le L(-) malate, le cis-aconitate, le trans aconitate, le 2-céto-D-gluconate, le N-acétyl-glucosamine, le quinate, la bétaïne, le succinate, le fumarate, le glycérate, et le glucosamine.

Parmi les milieux d'entretien possibles de la souche, le milieu d'entretien du type MY agar de Difco (référence 0712-01-8) est considéré comme particulièrement avantageux: Ledit milieu MY agar de Difco a la composition suivante :
□ extrait de bacto-levure 3 g
□ extrait de malt 3 g
□ □ bacto-peptone 5 g
□ bacto-dextrose 10 g
□ bacto-agar 20 g

Pour la conservation de la souche, il est préférable de prévoir au moins une étape de préculture. Par étape de préculture, on entend une étape qui consiste à développer et multiplier la souche bactérienne, sans production de polysaccharide.

Il a pu être mis en évidence que d'une manière générale, l'hétéropolysaccharide (HP) comporte des motifs de glucose et/ou ses dérivés, de galactose et/ou ses dérivés, de l'acide mannuronique et/ou ses sels, de l'acide acétique et/ou ses sels.

Les motifs constitutifs de l'hétéropolysaccharide (HP) sont en général présents dans des proportions molaires suivantes, en prenant comme référence le galactose égal à 1 :
- glucose et/ou ses dérivés 0,2 - 5,
- acide mannuronique et/ou ses sels 0,2 - 5,
- acide acétique et/ou ses sels 0 - 10,

Plus particulièrement, lesdits motifs sont présents dans des proportions molaires suivantes, en prenant comme référence le galactose égal à 1 :
- glucose et/ou ses dérivés 0,5 - 4, et de préférence 0,8 - 2,
- acide mannuronique et/ou ses sels 0,5 - 4 et de préférence 0,8 - 2,
- acide acétique et/ou ses sels 0 - 8, et de préférence 0 - 6,

Les acides mannuronique et acétique peuvent se présenter sous forme de sels. A titre de sels on peut citer les sels de sodium, de potassium, de calcium ou d'ammonium.

Les méthodes d'analyse de l'hétéropolysaccharide (HP) qui ont permis de déterminer sa formule brute telle que spécifiée ci-dessus, ont pour principe la détermination des éléments constitutifs (monosaccharides et acides) après hydrolyse dudit hétéropolysaccharide (HP) et dosages chromatographiques par étalonnage inteme ou exteme.

Ainsi, le dosage des monosaccharides a été réalisé de la manière suivante : 100 mg d'hétéropolysaccharide (HP) sont hydrolysées en tubes hermétiques par 5 ml d'acide trifluoroacétique molaire à 105°C pendant trois à six heures.

Cette opération est suivie d'une évaporation à sec et d'une reprise du résidu sec dans 5 ml de pyridine contenant 15 mg de sorbitol en tant qu'étalon interne ; puis une silylation sur 1 ml de solution pyridinique par 0,9 ml d'hexaméthyldisilazane. La silylation est catalysée par 0,1 ml d'acide trifluoroacétique.

Le dosage des monosaccharides est ensuite effectué par chromatographie en phase gazeuse à détection F.I.D. (Flame Ionisation Detection), sur colonne capillaire en verre de 25 mètres de longueur et de 0,25 mm de diamètre, chargée de phase méthyisilicone présentant une épaisseur de film de 0,14 microns. Le gaz vecteur utilisé est l'hydrogène, avec débit de 2 ml/minute.

Le dosage de l'acide acétique se fait après hydrolyse de 100 mg d'hétéropolysaccharide (HP) par 5 ml d'acide chlorhydrique 2N à 105 °C pendant une heure. Puis on ajoute 5 ml d'une solution d'acide propionique à 5 mg/ml en tant qu'étalon interne et on complète par 15 ml d'eau déminéralisée. Le dosage est effectué par CLHP au moyen d'une colonne de silice greffée C-18 de 5 microns de longueur 250 cm et de diamètre 4,6 mm. L'éluant est une solution aqueuse d'acide phosphorique 0,02 mol/l à un débit de 1,2 ml/minute. La détection est réfractométrique.

L'acide mannuronique est dosé par l'intermédiaire du CO₂ libéré par la décarboxylationsuite au traitement à chaud de la gomme par de l'acide chlorhydrique selon la méthode décrite dans le Food Chemical Codex, 4ème édition, page 768.

La masse molaire en poids est déterminée par chromatographie d'exclusion sur colonne TSK PW 4000 et 6000 en série (colonnes de longueur 30 cm et de diamètre 7 mm), avec détection réfractométrique. L'éluant est une solution de nitrate de sodium 0,1 mol/l. L'hétéropolysaccharide est à environ 0,015 % en poids dans l'éluant. L'étalonnage est réalisé au moyen de pullulanes qui sont des polysaccharides monodisperses de masses molaires comprises entre 5.10³ et 1,6.10⁶ g/mol extrapolés jusqu'à 10⁷ g/mol.

La masse molaire moyenne en poids (Mw) est obtenue à partir de la courbe de distribution massique issue du chromatogramme ; elle est généralement comprise entre 1.10⁵ et 8.10⁶ g/mol, de préférence comprise environ entre 8.10⁵ et 5.10⁶ g/mol.

Plus paticulièrement (HP) présente une masse molaire moyenne en poids (Mw) comprise environ entre 2,5.10⁶ et 4.10⁶ g/mol, les bomes étant inclues.

Comme déjà mentionné le (HP) présente de très bonnes propriétés rhéologiques en solution notamment dans l'eau distillée ou l'eau de ville.

Ainsi, on a pu constater que par exemple des solutions à 0,5 % en poids/poids de (HP) dans l'eau distillée à 23°C, et à une fréquence de 1 Hz, conduisent à des valeurs de G' comprises entre 0.1 et 200 Pa, et de G" comprises entre 0.1 et 20 Pa.

(HP) conduit à des gels forts ou vrais lorsque les valeurs de G' et G" sont comprises avantageusement entre 20 et 200 Pa pour G', et entre 0,5 et 15 Pa pour G". Plus avantageusement encore. G' est compris entre 20 et 150 Pa, et G" entre 0.5 et 10 . Pa. Selon un mode particulièrement préféré, la valeur de G' est d'environ 100 Pa et celle de G" est d'environ 5 Pa (dans l'eau distillée).

Le (HP) confère au milieu aqueux de la viscosité qui est évaluée par rhéologie en écoulement. Les mesures rhéologiques de viscosité en écoulement sont réalisées au moyen de rhéomètre à contrainte imposée ou à gradient de cisaillement imposé, comme par exemple au moyen d'un viscosimètre de type respectivement RHEOMAT® ou CARRIMED®.

Dans les deux cas, l'appareillage mesure la contrainte à l'écoulement du mélange HP + eau lorsque ce mélange est déformé irréversiblement. A partir de la contrainte se calcule la viscosité en écoulement.

Cet appareillage permet ainsi de quantifier le niveau de viscosité à un gradient de cisaillement donné.

La viscosité en écoulement peut être plus simplement évaluée à l'aide d'un viscosimètre BROOKFIELD®.

Ces mesures rhéologiques de viscosité en écoulement de (HP) permettent. de plus, d'évaluer le seuil d'écoulement de la solution de (HP) et/ou de la formulation le comprenant. Ledit seuil représente la force à fournir pour détruire la structure du milieu et le forcer à s'écouler.

La rhéologie en écoulement permet également de quantifier la facilité d'une solution de (HP) et/ou d'une formulation le comprenant à s'écouler lorsque le cisaillement imposé augmente (comportement pseudo-plastique ou rhéofluidifiant).

On a constaté par exemple que des solutions à 1 % en poids/poids de HP dans l'eau distillée contenant 0,5 % poids/poids de NaCl, à 23°C, conduit à des valeurs de viscosité en écoulement à gradient de cisaillement de 0,1 s⁻¹ comprises entre 100 et 5000 Pa.s, et plus particulièrement entre 200 et 2000 Pa.s.

Dans des conditions similaires, à un gradient de cisaillement de 10 s⁻¹ comprises entre 0,5 et 300 Pa.s, et plus particulièrement entre 5 et 150 Pa.s.

Ces données de rhéologie en écoulement sont représentatives du comportement de la formulation lors de sa mastication, lors de son transvasement, de son foisonnement etc.

Les gels obtenus par incorporation de (HP) dans le milieu, sont des gels cicatrisants, c'est-à-dire qu'après un cisaillement, même fort, les gels "fracturés" ont le pouvoir de se reformer et de retrouver leurs propriétés initiales.

Le pouvoir cicatrisant des gels obtenus à partir de (HP) est évalué par des mesures de compressométrie effectuées par exemple sur un texturant ETIA T2 composé d'un corps de mesure cylindrique de 12,7 mm de diamètre, une vitesse de pénétration de 0,05 mm/s, et une hauteur d'enfoncement de 15 mm. Le piston est enfoncé dans le gel au même endroit plusieurs fois, à des intervalles de temps différents, et on enregistre la force de compression. On détermine la pente à l'origine exprimée en mN/mm, représentative de l'élasticité du gel.

Par exemple un gel est préparé avec 0.5 % en poids/poids de (HP) dans l'eau distillée. Ce gel est ensuite stocké 24 heures avant d'effectuer les mesures de compressométrie, soit à température ambiante ( environ 25°C). soit à froid à environ 6°C.

Des mesures de compressométrie sont effectuées à des intervalles de temps différents : 0, 5, 15 minutes et 24 heures, avec une attente de 5 minutes entre chaque mesure.

Ainsi, la pente demeure constante est environ égale à 45 ± 1 mN/mm, quel que soit le temps de mesure (t= 0, 5, 15 minutes et 24 heures).

Ceci signifie que l'élasticité du gel est stable et qu'il a le pouvoir de se cicatriser plusieurs fois de suite au cours du temps tout en maintenant la même force de gel.

La présente invention a également trait à un procédé de préparation de l'hétéropolysaccharide (HP) tel que défini précédemment.

Le procédé de préparation consiste d'abord en la fermentation d'un milieu comportant au moins une source de carbone assimilable, par une souche *Pseudomonas sp I - 2054* (ou *DSM 12295*), un de ses recombinants ou un de ses mutants.

Outre ladite source de carbone assimilable, le milieu de fermentation peut aussi renfermer au moins une source d'azote organique ou minérale, et éventuellement un ou plusieurs sels minéraux.

Le milieu est inoculé de manière classique par la souche *Pseudomonas I - 2054* (ou *DSM 12295*).

A titre de source organique de carbone constitutive du milieu de fermentation, outre les sucres cités précédemment, on peut aussi citer des sucres tels que l'amidon avantageusement hydrolysé, les hydrolysats d'amidon, les mélanges de ces sucres, et les mélanges comprenant au moins un de ces sucres.

Plus particulièrement, on peut citer le glucose, le saccharose, l'amidon avantageusement hydrolysé, les hydrolysats d'amidon, le lactose, les mélanges de ces sucres, et les mélanges comprenant au moins un de ces sucres. Le glucose et le saccharose sont les sucres encore plus préférés.

La concentration en source de carbone dans le milieu de fermentation peut être comprise entre 1 et 100 g/l, et de préférence entre 15 et 60 g/l.

A titre de source organique d'azote, on peut citer la caséine et les caséinates, les hydrolysats de poisson, les farines de blé, de maïs, ou de soja, les extraits de levure (levure de boulanger, levure de bière, levures lactiques, etc.), com steap liquor (CSL), l'urée, et les protéines de pomme de terre.

A titre de sources minérales d'azote, on peut citer les nitrates d'ammonium ou de sodium, les phosphates ou les sulfates, d'ammonium.

La fermentation peut aussi avoir lieu avec un mélange de sources d'azote organiques et minérales.

La concentration en source azotée (organique, minérale, ou mélange des deux) dans le milieu de fermentation peut être comprise entre 1 et 80 g/l, et de préférence entre 3 et 50 g/l.

Le milieu de fermentation renferme avantageusement du calcium, seul ou éventuellement en mélange avec d'autres oligo-éléments tels que du fer, du manganèse et/ou du magnésium, ainsi que des vitamines et des nucléotides.

Le calcium peut être introduit dans le milieu sous forme d'une composition ou d'un composé minéral(e) ou organique, comme par exemple du CSL, de la farine de soja, des sels de phosphate, nitrate, carbonate, sulfate.

La fermentation peut être réalisée à des pressions comprises entre 1 et 4 bar à une température comprise entre 25°C et 35°C, de préférence entre 25°C et 30°C, dans des conditions aérobies.

Le pH du milieu de fermentation peut être compris entre 5 et 9, et de préférence entre 6 et 8. Le pH peut être ajusté, selon le cas, avec une base telle que la soude, la potasse, ou l'ammoniaque, ou avec un acide tel que l'acide sulfurique, l'acide phosphorique, l'acide chlorhydrique ou l'acide nitrique.

Le milieu de fermentation, placé dans une cuve ou un récipient de fermentation, peut être avantageusement soumis à une agitation. Cette agitation peut être exercée par exemple au moyen d'un secoueur réciproque, d'un secoueur giratoire, d'un mobile d'agitation ou d'une colonne à bulles. Le temps de fermentation est habituellement supérieur à 30 heures, mais généralement compris entre 40 et 100 heures.

Les rendements de fermentation sont généralement supérieurs à 40 %, plus particulièrement compris entre 55 et 75 %, et tout particulièrement compris entre 60 et 75 % en poids d'hétéropolysaccharide (HP) produit par rapport à la source de carbone mise en oeuvre.

Après la fermentation, l'hétéropolysaccharide (HP) peut être séparé du moût de fermentation selon les étapes suivantes :
*i* - on soumet le moût de fin de fermentation à un traitement thermique entre 80°C et 120 °C pendant environ 10 à 60 minutes,
*ii* - on précipite le hétéropolysaccharide (HP) au moyen d'un liquide organique au moins partiellement miscible avec l'eau,
*iii -* on sépare l'hétéropolysaccharide (HP) du liquide organique.

Dans l'étape *(i)*, le moût de fermentation renfermant l'hétéropolysaccharide (HP) est avantageusement chauffé à des températures comprises entre 80°C et 120°C, pendant 10 à 60 minutes, et de préférence entre 15 et 45 minutes.

Le moût soumis au traitement thermique ci-dessus présente avantageusement un pH compris entre 6 et 8.

Cependant, ce pH peut être ajusté si nécessaire, selon le cas, avec une base ou un acide.

Ces derniers peuvent être choisis parmi les bases et les acides mentionnés ci-dessus utilisés pour l'ajustement du pH du milieu de fermentation.

Selon une variante préférée de l'invention, le moût issu de l'étape (i) est maintenu à la même température que la température du traitement thermique.

Dans l'étape *(ii)*, on récupère l'hétéropolysaccharide (HP) du moût obtenu dans l'étape (i) avantageusement par précipitation au moyen d'un liquide organique au moins partiellement miscible avec l'eau et dans lequel l'hétéropolysaccharide (HP) est insoluble ou pratiquement insoluble.

A titre de liquides convenables selon la présente invention, on peut citer l'acétone ou les alcools comportant de 1 à 6 atomes de carbone tels que l'éthanol, le propanol, l'isopropanol, le butanol, le tertio-butanol, ou leur mélange.

Plus particulièrement la précipitation de (HP) est effectuée avec l'isopropanol.

Le volume de liquide organique utilisé est généralement au moins 2 fois celui du volume de moût à traiter.

La précipitation de l'hétéropolysaccharide (HP) par un liquide organique peut également être réalisée en présence de sels, tels que les sulfates, les chlorures, ou les phosphates, de sodium, de potassium, ou de calcium.

Selon un mode de réalisation particulier, la précipitation peut avoir lieu à une température comprise entre 40 et 60°C.

L'hétéropolysaccharide (HP) une fois précipité, peut ensuite être séparé, dans l'étape *(iii)*, du liquide organique.

La méthode de séparation n'est pas critique en soi et peut être choisie indifféremment parmi les méthodes de séparation usuelles connues comme par exemple la filtration. la centrifugation ou l'essorage.

Les fibres obtenues peuvent être facultativement déshydratées par exemple au moyen d'acétone ou d'un alcool tel que l'éthanol, le propanol, ou l'isopropanol.

Le poids d'alcool nécessaire pour effectuer cette opération de déshydratation est généralement de 1 à 10 fois celui des fibres à traiter.

Les fibres déshydratées peuvent subir de nouvelles opérations de filtration, de centrifugation ou d'essorage.

Le cas échéant, les fibres peuvent être séchées, broyées et/ou tamisées de façon à obtenir une poudre d'hétéropolysaccharide (HP).

Si l'on souhaite obtenir une poudre plus pure, il est possible de traiter soit le moût de fermentation, soit une solution aqueuse reconstituée à partir de la poudre obtenue selon le procédé décrit ci-dessus, au moyen d'une ou plusieurs enzymes.

A titre d'enzymes pouvant convenir à cet effet, on peut citer les protéases, les mutanases, les lipoprotéases, les cellulases, et les chitinases.

La purification enzymatique peut être associée ou remplacée par des procédés physiques de purification tels que les divers modes de filtration, de centrifugation. de dialyse, ou par différentes techniques de chromatographies.

Les moûts de fermentation et les solutions reconstituées d'hétéropolysaccharide (HP), ayant subi ou non un traitement de purification, peuvent être concentrés.

La concentration peut être avantageuse dans certains cas, en particulier lorsque les coûts de transport peuvent être ainsi réduits. De plus, les solutions concentrées peuvent être plus rapidement mises en oeuvre que les poudres d'hétéropolysaccharide (HP).

La concentration peut être réalisée par toutes les techniques connues par l'homme du métier, notamment l'évaporation, l'ultrafiltration, ou la diafiltration.

Dans la présente invention, l'hétéropolysaccharide (HP) est présent avantageusement sous la forme d'un solide de type fibre ou poudre.

Comme déjà mentionné, (HP) présente de très bonnes propriétés rhéologiques et notamment la faculté de former des gels vrais. Selon les conditions de fermentation, en particulier selon les composants et leurs concentrations dans le milieu de culture, et/ou les conditions de précipitation dans l'étape (ii) du procédé (plus particulièrement si la précipitation se fait ou non en présence de sels), (HP) a l'avantage de pouvoir être utilisé soit en tant qu'agent épaississant soit en tant qu'agent gélifiant, soit les deux.

Ainsi, la présente invention concerne l'utilisation de l'hétéropolysaccharide (HP) tel que décrit précédemment ou tel qu'obtenu par le procédé défini ci-dessus, en tant qu'agent épaississant et/ou gélifiant.

(HP) peut être utilisé en tant qu'agent épaississant et/ou gélifiant par exemple dans les industries pétrolière, agrochimique, alimentaire, cosmétique, papetière, textile, ainsi que dans les peintures, les lentilles de contact, les colles, les encres, et les nettoyants ménagers ou industriels.

La quantité d'hétéropolysaccharide (HP) de l'invention pouvant être mise en oeuvre dans des compositions cosmétiques est fonction du milieu aqueux à épaissir et/ou à gélifier. Celle-ci peut représenter de 0,01 % à 5 % environ, de préférence de l'ordre de 0,1 % à 0.3% du poids du milieu aqueux épaissi ou gélifié.

On entend par le terme composition ou formulation cosmétique tous les produits ou préparations cosmétiques du type de ceux ou celles décrit(e)s dans l'annexe I ("Illustrative list by category of cosmetic products") de la directive européenne n° 76/768/CEE du 27 juillet 1976, dite directive cosmétique.

Les compositions cosmétiques peuvent être formulées en un grand nombre de types de produits pour la peau et/ ou le cheveu, comme les mousses, les gels (coiffants notamment), les conditionneurs, les formulations pour le coiffage ou pour faciliter le peignage des cheveux, les formules de rinçage, les lotions pour les mains et le corps, les produits régulant l'hydratation de la peau, les laits de toilette, les compositions démaquillantes. les crèmes ou lotions de protection contre le soleil et le rayonnement ultra-violet, les crèmes de soins, les préparations anti-acnée, les analgésiques locaux, les mascaras, les produits destinés à être appliqués sur les lèvres ou autres muqueuses, les sticks, et bien d'autres compositions du même type.

Ces compositions cosmétiques font appel à un véhicule, ou à un mélange de plusieurs véhicules, présent dans lesdites compositions à des concentrations comprises entre 0,5 % et 99.5 % environ, généralement entre 5 et 90 % environ.

Le choix du véhicule approprié dépend de la nature des ingrédients utilisés, et de la destination desdites compositions, selon que le produit formulé est censé être laissé sur la surface où il a été appliqué (par exemple sprays, mousses, lotion tonique, ou gels) ou au contraire rincé après utilisation (par exemple shampoing, conditionneur, lotions de rinçage).

Les véhicules aqueux présents dans les compositions cosmétiques peuvent contenir en outre des alcools en C₁-C₆, en particulier le méthanol. l'éthanol, l'isopropanol. Ils peuvent également contenir un autre solvant permettant de solubiliser ou de disperser, dans le milieu aqueux, les divers ingrédients utilisés dans lesdites compositions.

Lesdits véhicules peuvent ainsi contenir en outre une grande variété d'autres solvants comme l'acétone, les hydrocarbures, les hydrocarbures halogénés, le linalol, les esters et les silicones volatils. Les différents solvants pouvant être utilisés dans les véhicules aqueux peuvent être miscibles ou non miscibles les uns avec les autres.

Lorsque les compositions cosmétiques se présentent sous la forme de sprays, lotions toniques, gels ou mousses, les véhicules préférentiels comprennent à côté de l'eau, de l'éthanol, des dérivés volatils de silicone, et leurs mélanges.

Les formulations pour mousses et sprays aérosol peuvent aussi renfermer un propulseur capable de générer les produits sous forme de mousse ou de sprays fins, uniformes. A titre d'exemples, on peut citer le trichlorofluorométhane, le dichlorodifluorométhane, le difluoroéthane, le diméthyléther, le propane, le n-butane ou l'isobutane.

Lesdits véhicules aqueux peuvent prendre un grand nombre de formes, notamment celles d'émulsions, incluant les émulsions eau dans huile, huile dans eau, et émulsions multiples, dont la viscosité recherchée peut aller jusqu'à 2 000 000 mPa.s.

A côté du véhicule aqueux. les compositions cosmétiques peuvent contenir des agents tensioactifs, mis en oeuvre pour disperser, émulsionner, solubiliser, stabiliser divers composés utilisés notamment pour leurs propriétés émollientes ou humectantes. Ils peuvent être de type anionique, non-ionique, cationique, zwitterionique ou amphotère ; on peut citer à titre d'exemples :
□ des agents tensio-actifs anioniques en quantité pouvant aller de 3 % à 50 %, de préférence de 5 % à 20 %, agents tels que
   . les alkylesters sulfonates
   . les alkylsulfates
   . les alkylamides sulfates
   . les sels d'acides gras saturés ou insaturés
□ agents tensio-actifs non-ioniques en quantité pouvant aller de 0,1 % à 30 %, de préférence de 2 % à 10 %, agents tels que
   . les alkylphénols polyoxyalkylénés
   . les glucosamides, glucamides ;
   . les glycérolamides dérivés de N-alkylamines
   . les alcools aliphatiques en C₈-C₂₂ polyoxyalkylénés
   . les produits résultant de la condensation de l'oxyde d'éthylène avec un composé hydrophobe résultant de la condensation de l'oxyde de propylène avec le propylène glycol,
   . les oxydes d'amines
   . les alkylpolyglycosides et leurs dérivés polyoxyalkylénés;
   . les amides d'acides gras en C₈-C₂₀
   . les acides gras éthoxylés
   . les amides, amines, amidoamines éthoxylées
□ agents tensio-actifs amphotères et zwitterioniques en quantité pouvant aller de 0.1 % à 30 %, de préférence de 1 % à 10 %, agents tels que
   * ceux de type bétaïne comme
      - les bétaïnes
      - les sulfo-bétaïnes
      - les amidoalkylbétaïnes
      - et les sulfo-bétaïnes
   * les alkylsultaines
   * les produits de condensation d'acides gras et d'hydrolysats de proteines,
   * les cocoamphoacétates et cocoamphodiacétates
   * les alkylampho-propionates ou -dipropionates,
   * les dérivés amphotères des alkylpolyamines

Peuvent également être présents des agents conditionneurs, en quantité pouvant aller de 0,05 % à 5 %, de préférence de 0,1 % à 1 %.

Parmi ceux-ci, on peut mentionner ceux d'origine synthétique plus connus sous le nom polyquatemium comme les polyquatemiums -2,-7, et -10, les dérivés cationiques de polysaccharides, comme la cellulose cocodimonium hydroxyéthyl, le guar hydroxypropyl trimonium chlorure, l'hydroxypropyl guar hydroxypropyl trimonium chlorure, les dérivés non volatils de silicones comme l'amodiméthicone, les cyclométhicones, les organopolysiloxanes non hydrosolubles et non volatils comme les huiles, résines ou gommes telles que les gommes diphényldiméthicone.

Les compositions cosmétiques peuvent également contenir des polymères présentant des propriétés filmogènes pouvant être utilisés pour apporter une fonction fixante. Ces polymères sont généralement présents à des concentrations comprises entre 0,01 et 10%, préférentiellement entre 0,5 et 5 %. Ils sont préférentiellement du type polyvinylpyrrolidone, copolymères de polyvinylpyrrolidone et de méthyl méthacrylate, copotymère de polyvinylpyrrolidone et d'acétate de vinyle, copolymères polytéréphtale d'éthylène glycol / polyéthylène glycol, polymères copolyesters téréphtaliques sulfonés.

Les compositions cosmétiques peuvent également contenir des dérivés polymériques exerçant une fonction protectrice, en quantités de l'ordre de 0,01-10 %, de préférence environ 0,1-5 % en poids, dérivés tels que
. les dérivés cellulosiques
. les polyvinylesters greffés sur des troncs polyalkytenes
. les alcools polyvinyliques
. polymères copolyesters téréphtaliques sulfonés
. les monoamines ou polyamines éthoxylées, les polymères d'amines éthoxylées

Les performances des compositions cosmétiques peuvent aussi être améliorées par l'emploi d'agents plastifiants, en quantité pouvant aller de 0,1 à 20% de la formulation, de préférence de 1 à 15 %. Parmi ces agents, on peut citer les adipates, les phtalates, les isophtalates, les azélates, les stéarates, les silicones copolyols, les glycols, l'huile de ricin, ou leurs mélanges.

On peut aussi avantageusement ajouter à ces compositions des agents séquestrants des métaux, plus particulièrement ceux séquestrants du calcium comme les ions citrates, ou des agents dispersants polymériques en quantité de l'ordre de 0.1-7 % en poids, pour contrôler la dureté en calcium et magnésium, agents tels que
. les sels hydrosolubles d'acides polycarboxyliques
. les polyéthylèneglycols de masse moléculaire de l'ordre de 1000 à 50 000

On peut également incorporer aux compositions cosmétiques des agents humectants ; on peut citer le glycérol, le sorbitol, l'urée, le collagène, la gélatine, et des émollients qui sont généralement choisis parmi les alkylmonoglycérides, les alkyldiglycérides, les triglycérides comme les huiles extraites des plantes et des végétaux ou les huiles d'origine animale ou leurs dérivés hydrogénés, les huiles minérales ou les huiles paraffiniques, les diols, les esters gras, les silicones.

A ces composés, on peut ajouter en association des poudres ou des particules minérales comme du carbonate de calcium, des oxydes minéraux sous forme de poudre ou sous forme colloïdale comme du dioxyde de titane, de la silice, des sels d'aluminium. du kaolin, du talc, des argiles et leurs dérivés.

A ces ingrédients on rajoute généralement un ou des parfums, des agents colorants et/ou des agents opacifiants comme des pigments.

Pour protéger la peau et/ou les cheveux des agressions du soleil et des rayons UV, on peut ajouter à ces formulations des filtres solaires qui sont soit des composés chimiques absorbant fortement le rayonnement UV ou des particules minérales, comme l'oxyde de zinc, te dioxyde de titane ou les oxydes de cérium.

Des agents conservateurs comme les esters de l'acide p-hydroxybenzoïque. le benzoate de sodium, ou tout agent chimique évitant la prolifération bactérienne ou des moisissures et utilisé traditionnellement des les compositions cosmétiques sont généralement introduits dans ces compositions à hauteur de 0,01 à 3 % en poids.

On peut parfois utiliser des agents modifiant l'activité de l'eau et augmentant fortement la pression osmotique, comme les carbohydrates ou des sels.

La composition cosmétique peut aussi contenir des polymères viscosants et/ou gélifiants autres, comme les polyacrylates réticulés, les hydrocolloïdes obtenus par fermentation comme la gomme xanthane et le Rhéozan®, les dérivés de la cellulose comme l'hydroxypropylcellulose, la carboxyméthylcellulose, les guars et leurs dérivés.... utilisés seuls ou en association.

L'invention conceme plus particulièrement l'utilisation de l'hétéropolysaccharide en tant qu'agent épaississant et/ou gélifiant dans des formulations alimentaires.

Les formulations alimentaires dans lesquelles est ajouté l'hétéropolysaccharide (HP) sont classiquement des émulsions simples ou multiples de liquides, des émulsions complexes de gaz et de liquides (systèmes foisonnés), des suspensions de liquides et de solides, ou tout autre système combinant ces possibilités.

Dans ces formulations, le liquide est avantageusement l'eau ou un liquide comprenant au moins en partie de l'eau.

Les formulations alimentaires sont obtenues en mettant en oeuvre les méthodes classiques de préparation des formulations alimentaires selon leur type. Ainsi, le (HP) avantageusement sous la forme d'un solide de type fibre ou poudre est mélangé aux autres ingrédients nécessaires à la formulation. L'ensemble peut, le cas échéant, être homogénéisé.

La température à laquelle est préparée la formulation n'est pas en soi critique. Les formulations comprenant le (HP) peuvent être stérilisées sans aucun dommage pour leurs propriétés d'usage. Un autre avantage de (HP) est qu'il est possible de préparer les formulations alimentaires sans avoir à chauffer au préalable les ingrédients.

(HP) demeure compatible malgré la diversité des formulations alimentaires (pH, force ionique, composition), et conserve substantiellement ses propriétés.

Les propriétés rhéologiques intéressantes associées à l'hétéropolysaccharide (HP) objet de l'invention, ainsi que la faculté de ce dernier à conduire à des gels vrais à des températures inférieures ou égales à 40°C, et ce dans une large gamme de pH, permet, en outre, de conférer aux formulations dans lesquelles il est utilisé seul ou en mélange avec d'autres additifs, une texture voisine de celle des formulations comprenant exclusivement lesdits additifs.

Les paramètres mesurables pour caractériser la texture des formulations alimentaires sont de nature rhéologique, et consistent essentiellement à mesurer les modules élastique (G') et visqueux (G"), et la viscosité en écoulement à un gradient de cisaillement donné. G' et G", ainsi que la viscosité ont été précédemment définis.

Ces caractéristiques rhéologiques ont comme objectif de mettre en évidence les comportements visco-élastique et/ou pseudo-plastique des formulations, afin de les comparer entre elles.

(HP), avantageusement sous la forme d'un solide de type fibre ou poudre, a la faculté de conférer un profil rhéofluidifiant à la formulation le comprenant.

(HP) a, de même, la faculté de conduire à des gels vrais pouvant cicatriser après application d'une contrainte mécanique.

Il est à noter que les modules G' et G", ainsi que la viscosité mesurés pour une formulation peuvent être différents de ceux mesurés pour (HP) dans l'eau distillée.

Dans les desserts lactés et gélifiés comme par exemple les flans on peut avantageusement remplacer au moins partiellement les gélifiants usuels notamment la gélatine par (HP).

Dans les milieux salés-acides, comme les vinaigrettes, on peut structurer le milieu aqueux contenu par l'ajout de faibles quantités de (HP).

Dans le domaine de la confiserie notamment dans les bonbons gélifiés du type HARIBO®, on peut avantageusement remplacer au moins partiellement les gélifiants comme par exemple la gélatine, par (HP).

Dans les milieux à force ionique élevée notamment en charcuterie, (HP) peut être rajouté aux carraghénanes, pour renforcer la texture en particulier l'aspect élastique des saucisses par exemple.

Dans les formulations destinées à être foisonnées, comme les crèmes chantilly. les nappages, les crèmes glacées, (HP) peut être employé comme. agent épaississant et/ou gélifiant.

(HP) peut de même être utilisé dans des formulations comme les mayonnaises, les mousses de légumes, les mousses comprenant des protéines, telles que les mousses de viande, de poissons, les mousses comprenant de l'albumine comme les meringues.

En tant qu'agent épaississant et/ou gélifiant, (HP) peut également entrer dans la composition des yoghourts.

Dans les applications alimentaires précitées, on utilise en général de 0,01 à 5 % en poids, et de préférence entre 0,05 à 2 % en poids d'hétéropolysaccharide (HP) par rapport au poids de la composition ou formulation qui le renferme. Plus préférentiellement encore, on utilise 0.1 à 1 % en poids d'hétéropolysaccharide (HP) par rapport au poids de la composition ou formulation.

Il est à noter que l'hétéropolysaccharide (HP) n'altère pas le goût des aliments dans lesquels il est introduit.

L'invention concerne enfin les compositions ou les formulations alimentaires comprenant l'hétéropolysaccharide (HP) tel que défini précédemment.

Les exemples suivants illustrent la présente invention, sans toutefois en limiter la portée.

### EXEMPLES

### Exemple 1

Cet exemple décrit la culture pure de Pseudomonas *sp I - 2054* (ou *DSM 12295*), et les conditions de conservation de la souche.

### Culture pure de Pseudomonas sp I - 2054 (ou DSM 12295) :

Le milieu d'entretien de la souche *Pseudomonas sp I - 2054* ( ou *DSM 12295*) est le milieu MY agar de Difco (référence 0712-01-8). La composition de ce milieu, déjà prêt, est :
□ extrait de bacto-levure 3 g
□ extrait de malt 3 g
□ bacto-peptone 5 g
□ bacto-dextrose 10 g
□ bacto-agar 20 g

On dilue 21 g de ce milieu dans un litre d'eau distillée. Après dissolution, le milieu est stérilisé à l'autoclave pendant 15 minutes à 121°C. On répartit ensuite le milieu en boîtes de Pétri.

La culture est réalisée sur boîte de Pétri incubée entre 25°C et 30°C. de préférence entre 25°C et 28 °C, pendant 24 heures minimum.

### Préculture - conservation :

La souche est alors conservée sous la forme de tube congelé à -196°C par le procédé de congélation azote liquide (CAL).

Pour une congélation azote liquide (CAL) on réalise une préculture sur milieu PYG10 ayant la composition suivante :
□ extrait de malt 3 g (procuré auprès de Oxoïd)
□ extrait de levure 3 g (Oxoïd)
□ peptone de soja 5 g (Oxoïd)
□ glucose 10 g (procuré auprès de Prolabo)
□ eau de source qsp 1 I.

Pour la préparation du milieu, tous les ingrédients sont dispersés dans l'eau de source. Le pH est ajusté à 6,5 avec H₂SO₄ 10 %. Le milieu est stérilisé 20 minutes à 120°C. à l'autoclave.

Après 24 heures d'incubation à 28°C sur secoueur giratoire à 220 tr/min et amplitude = 50 mm, 10 % en volume de glycérol pur stérile sont ajoutés à la culture. La culture est ensuite répartie dans des cryotubes de contenances variant de 1 ml à 10 ml, de préférence de 2 ml à 4 ml.

Ces tubes sont conservés dans l'azote liquide.

### Exemple 2

Cet exemple décrit la préparation et l'obtention de l'hétéropolysaccharide selon deux procédés de fermentation, l'un avec une source d'azote organique et l'autre avec une source d'azote minérale.

Dans cet exemple deux étapes de "préculture" interviennent. Ces étapes ont lieu en erienmeyers de 500 ml, ce qui correspond à 100 ml de milieu.

L'étape de production qui correspond à l'étape au cours de laquelle la souche bactérienne produit le polysaccharide, a lieu en fermenteur de 20 litres, dont 15 litres utiles.

Les conditions d'agitation du secoueur giratoire sont : vitesse = 220 tr/min et amplitude = 50 mm.

### Etape préculture 1 :

L'étape de préculture 1 est réalisée avec un milieu PYG 10 de composition suivante :
□ extrait de malt 3 g (Oxoïd)
□ extrait de levure 3 g (Oxoïd)
□ bacto-peptone 5 g (Oxoïd)
□ glucose 10 g (Prolabo)
□ eau distillée qsp 1 I.

Tous les ingrédients sont dispersés dans qsp 1 I d'eau distillée. Le pH est ajusté avant stérilisation à 6,5 avec H₂SO₄ 10 %. Le milieu est stérilisé 20 minutes à 120°C à l'autoclave.

Après stérilisation et avant inoculation par le cryotube (qsp). le pH est à 7,33.

Chaque erlenmeyer est ensemencé avec la quantité qsp de la CAL.

Après 24 heures d'incubation à 28°C sur secoueur giratoire (220 tr/min, A = 50 mm), le milieu présente les caractéristiques suivantes :
□ pH = 6,50
□ viscosité < 10 mPa.s
□ la population lue sur MY agar (milieu Difco, référence 0712-01-8) après 72 heures à 28°C = 1,7.10⁵ ufc/ml

Après 24 heures d'incubation, la préculture 1 est utilisée pour ensemencer la préculture 2.

### Etape de préculture 2 :

L'étape de préculture 2 est réalisée avec un milieu de composition suivante :
□ extrait de levure 4 g (Oxoïd)
□ MgSO₄,7H₂O 0,8 g (Prolabo)
□ FeSO₄,7H₂O 0.01 g (Prolabo)
□ MnSO₄,H₂O 5 ppm Mn²⁺ (Prolabo)
□ K₂HPO₄ 4 g (Prolabo)
   ou Na₂HPO₄ 3 g (Prolabo)
□ glucose 10 g (Prolabo)
□ eau adoucie qsp 1 l

Une solution de glucose à 100 g/l est préparée dans de l'eau distillée puis stérilisée à pH naturel 15 minutes à 121°C.

Le reste des ingrédients est dispersé dans qsp 900 ml d'eau adoucie puis ajusté à pH 6,8 avant la stérilisation 15 minutes à 121°C.

Après stérilisation, on ajoute 10 ml de la solution de glucose dans chaque erenmeyer.

Après stérilisation et avant inoculation, le pH est de 6,88.

Chaque erienmeyer est inoculé avec la quantité suffisante pour de la préculture 1.

Après 24 heures d'incubation à 28°C sur secoueur giratoire (220 tr/min, A = 50 mm), le milieu présente les caractéristiques suivantes :
□ pH = 6,82
□ viscosité = 50 - 100 mPa.s
□ la population lue sur MY agar (milieu Difco, référence 0712-01-8) après 72 heures à 28°C = 1,6.10⁹ ufc/ml

Après 24 heures d'incubation, la préculture 2 est utilisée pour ensemencer la les deux milieux de fermentation (fermenteurs 1 et 2) dans l'étape de production.

### Etape de production :

La dernière étape est l'étape de production de l'hétéropolysaccharide (HP). Le milieu du fermenteur 1 a la composition suivante :
□ glucose 20 g (Prolabo)
□ CSL (com-steep liquor) 6 g (Prolabo)
□ MgSO₄,7H₂O 0.8 g (Prolabo)
□ MnSO₄,H₂O 5 ppm Mn²⁺ (Prolabo)
□ K₂HPO₄ 1 g (Prolabo)
□ antimousse 0,2 ml
□ eau adoucie qsp 1 l
Glucose 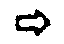 Qsp grammes de glucose sont dissous dans qsp 3 I d'eau adoucie. Le pH est abaissé à 5 par H₂SO₄ 10 %. La solution est stérilisée en flacon de Mariotte 30 minutes à 120°C à l'autoclave.
Azote + sels 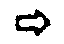 Qsp grammes de com-steep liquor (CSL), 15 g de K₂HPO₄, 12 g de MgSO₄,7H₂O, 23 ml d'une solution de MnSO₄,H₂O à 10 g/l, et 3 ml d'antimousse sont dissous dans qsp 7 I d'eau adoucie. Le pH est ajusté à 6,5 avec H₂SO₄ 10 %. Ce mélange est stérilisé *in situ* 30 minutes à 120°C.
Soude 1N 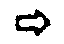 40 g de pastilles de NaOH sont dissous dans qsp 1I d'eau distillée. La solution est stérilisée en flacon de Mariotte 30 minutes à 120°C à l'autoclave.

Quand tous les ingrédients sont à 28°C, ils sont mélangés dans le fermenteur. Le fermenteur est ensuite inoculé avec qsp de préculture 2.

Les conditions de fermentation dans le fermenteur 1 sont les suivantes :
Agitation 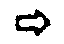 200 tr/min de 0 à 20 heures d'âge, puis 400 tr/min, jusquà la fin de la fermentation
Aération 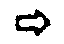 400 l/h de 0 à 18 heures puis 825 l/h de 24 heures jusqu'à la fin de la fermentation

La température est régulée à 28°C.
Le pH est régulé à 6,8 par NaOH 1N.
La pression est la pression atmosphérique.

Le milieu du fermenteur 2 a la composition suivante :
□ NaNO₃ 1,2 g (Prolabo)
□ NH₄NO₃ 0,25 g (Prolabo)
□ CaSO₄,2H₂O 0,3 g (Prolabo)
□ MgSO₄,7H₂O 0.8 g (Prolabo)
□ MnSO₄,H₂O 5 ppm Mn²⁺ (Prolabo)
□ FeSO₄,7H₂O 0,01 g (Prolabo)
□ Na₂HPO₄ 3 g (Prolabo)
□ glucose 45 g (Prolabo)
□ antimousse 0,2 ml
□ eau adoucie qsp 1 l
Glucose 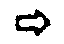 Qsp grammes de glucose sont dissous dans qsp 3 I d'eau adoucie. Le pH est ajusté à 5 par H₂SO₄ 10 %. La solution est stérilisée en flacon de Manotte 30 minutes à 120°C à l'autoclave.
Azote + sels 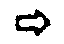 18 g de NaNO₃, 3,75 g de NH₄NO₃, 4,5 g de CaSO₄,2H₂O, 23 ml d'une solution de MnSO₄,H₂O à 10 g/l, 12 g de MgSO₄,7H₂O, 75 ml d'une solution de FeSO₄,7H₂O à 2 g/l, 4,5 g de Na₂HPO₄, et 3 ml d'antimousse sont dissous dans qsp 7l d'eau adoucie. Le pH de cette solution est ajusté à 6 avec H₂SO₄ 10 %. Ce mélange est stérilisé *in situ* 30 minutes à 120°C.
Soude 1N 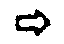 40 g de pastilles de NaOH sont dissous dans qsp 1l d'eau distillée. La solution est stérilisée en flacon de Mariotte 30 minutes à 120°C à l'autoclave.

Quand tous les ingrédients sont à 28°C, ils sont mélangés dans le fermenteur. Le fermenteur est ensuite inoculé avec qsp de préculture 2.

Les conditions de fermentation dans le fermenteur 2 sont les suivantes :
Agitation 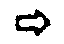 200 tr/min de 0 à 20 heures d'âge, puis 400 tr/min jusqu'à la fin de la fermentation
Aération 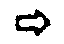 400 l/h de 0 à 24 heures puis 825 l/h de 24 heures jusqu'à la fin de la fermentation
La température est régulée à 28°C.
Le pH est régulé à 6,8 par NaOH 1N.
La pression est la pression atmosphérique.

### Résultats de fermentation :

Selon le milieu de culture étudié, les durées de fermentations varient de 60 à 115 heures, les matières sèches précipitables à l'isopropanol varient de 10 à 18 g/kg, et le rendement pondéral par rapport à la source de carbone mise en oeuvre varie de 44 à 70 %.

### Extraction et Purification

Le moût de fin de fermentation est stabilisé avec 10 % d'IPA pur (poids/poids). Il est ensuite traité thermiquement pendant 20 minutes à 100-110°C à pH 7. Le pH, pendant le traitement thermique, ne varie pas.

Sorti du traitement thermique, le moût est extrait à chaud (température > 70°C).

Les conditions de précipitation sont :
□ 1,7 kg de moût chaud dans 4,5 kg d'IPA pur (à 50°C environ).

Après la précipitation, les fibres sont hâchées puis lavées et déshydratées avec l'IPA ayant un titre de 78 %.

Les fibres sont ensuite séchées à l'étuve ventilée à environ 85°C jusqu'à obtention d'un produit ayant une humidité d'environ 10 % en poids.

Les fibres sont ensuite broyées et tamisées.

L'analyse des motifs de l'hétéropolysaccharide obtenu dans le milieu du fermenteur 1 (milieu organique) est comme suit en proportions molaires (pourcentage massique) :

| | | |
|---|---|---|
| Galactose | 1 | (13%) |
| Glucose | 1,08 | (14%) |
| Acide mannuronique | 1,1 | (17%) |
| Acide acétique | 4,3 | (18,6%) |

L'analyse des motifs de l'hétéropolysaccharide obtenu dans le milieu du fermenteur 2 (milieu minéral) est comme suit (pourcentage massique) :

| | |
|---|---|
| Galactose | (22,5%) |
| Glucose | (21,7%) |
| Acide mannuronique | (26%) |
| Acide acétique | (31%) |

### Exemple 3

Cet exemple a pour objet l'utilisation de (HP) obtenu dans l'exemple 2, dans une formulation alimentaire pour nappage.

Dans les exemples qui vont suivre, les viscosités en écoulement, exprimées en mPa.s, ont été mesurées au moyen d'un viscosimètre BROOKFIELD RVT 20-2, à température ambiante.

Les valeurs des modules élastiques, exprimées en Pa. ont été effectuées au moyen d'un rhéomètre CARRIMED CSL 100, à contrainte imposée. Elles ont été mesurées en régime oscillatoire - fréquence de 0,01 à 10 Hz.

Les mesures de taux de foisonnement, exprimées en %, ont été réalisées de la façon suivante :
- dans un bécher de volume (V) et de masse connus, on introduit de la mousse, on donne trois coups secs, et on arase ;
- on pèse le bécher pour déterminer la masse (M) de mousse qu'elle contient ;
- le taux de foisonnement = [ M(g) / V (ml) ] x 100

On prépare deux formulations :
formulation 3.1 : comprenant l'hétéropolysaccharide (HP) selon l'invention,
formulation 3.2 (comparative) : comprenant le caséinate de sodium, et l'alginate de sodium.
Les compositions des formulations sont récapitulées dans le Tableau I.

**Tableau I**

| **Composantes** | **Formulation 3.1 (% en poids)** | **Formulation 3.2 (% en poids)** |
|---|---|---|
| Phase huileuse | | |
| □ Huile de palme hydrogénée | 7,6 | 7,6 |
| □ Ester acétique de mono-diglycérides | 0,76 | 0,76 |
| □ Ester lactique de mono-digtycérides | 0,76 | 0,76 |

| Phase aqueuse | | |
|---|---|---|
| □ Sucre | 8,35 | 8,35 |
| □ Poudre de lait écrémé | 7,44 | 7,44 |
| □ Maltodextrine (Glucidex® 19) | 4,6 | 4,6 |
| □ Caséinate de sodium | - | 1,5 |
| □ Alginate de sodium | - | 0,02 |
| □ Hétéropolysaccharide (HP) | 0,5 | - |
| □ Eau | qsp 100 | qsp 100 |

### Phase aqueuse

Dans un bécher équipé d'une pale défloculeuse, on pèse la quantité d'eau requise et l'on disperse sous vive agitation (500 tr/min) le mélange de poudres décrit dans le tableau ci-dessus.

L'agitation est maintenue 5 minutes après l'introduction desdites poudres.

### Phase huileuse

Dans un bécher, on chauffe au bain-marie à 70°C la matière grasse et les émulsifiants.

On ajoute ensuite la phase huileuse à la phase aqueuse sous agitation à 1000 tr/min.

L'agitation est maintenue 5 minutes après l'introduction de la phase huileuse. Pendant cette opération, on compense l'évaporation de l'eau.

Le tout est ensuite homogénéisé à l'Ultra-turrax pendant 2 minutes à 20000 tr/min.

On refroidit le mélange à une température inférieure à 10°C, avant de procéder au foisonnement. Celui-ci a lieu en utilisant un mélangeur de laboratoire de type KENNWOOD CHEF à vitesse maximale pendant 3 minutes à une température proche de 5°C.

Les résultats sont rassemblés dans le Tableau II.

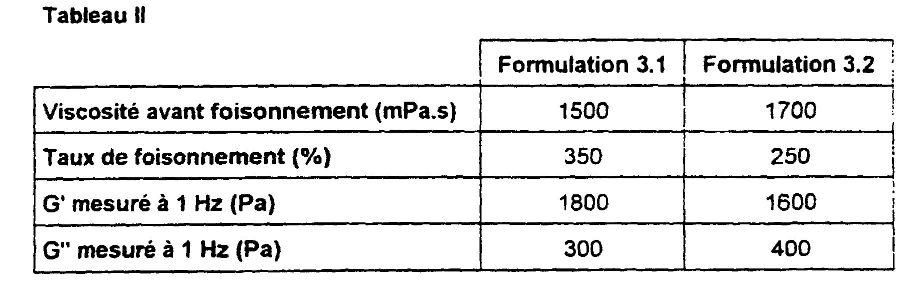

Ces résultats montrent que la formulation 3.1 mettant en oeuvre le (HP) selon l'invention, présente une viscosité plus faible que celle de la formulation 3.2 comparative, et de ce fait est plus facile à faire foisonner.

En outre, la formulation 3.1 (selon l'invention), est d'une part plus gélifiée (G' plus élevé à haute fréquence), et présente un taux de foisonnement amélioré.

## Revendications

1. Hétéropolysaccharide (HP) **caractérisé en ce qu'**il est susceptible d'être obtenu par fermentation d'un milieu comportant au moins une souche *Pseudomonas sp I* - *2054* (ou *DSM 12295*), un de ses recombinants, ou de ses mutants, et une source de carbone assimilable par ladite souche, l'un de ses recombinants, ou l'un de ses mutants.

2. Hétéropolysaccharide (HP) selon la revendication 1, **caractérisé en ce qu'**il comporte des motifs de glucose, de galactose, et/ou leurs dérivés, de l'acide mannuronique et/ou ses sels, et dont certains hydroxyles saccharidiques peuvent être estérifiés sous forme d'acétate.

3. Hétéropolysaccharide (HP) selon la revendication précédente, **caractérisé en ce qu'**il comporte lesdits motifs dans des proportions molaires suivantes, en prenant comme référence le galactose égal à 1 :
- glucose et/ou ses dérivés 0,2 - 5,
- acide mannuronique et/ou ses sels 0,2 - 5,
- hydroxyles saccharidiques estérifiés en acétate 0 - 10,

4. Hétéropolysaccharide (HP) selon l'une quelconque des revendications précédentes,
**caractérisé en ce qu'**il comporte lesdits motifs dans des proportions molaires suivantes en prenant comme référence le galactose égal à 1 :
- glucose et/ou ses dérivés 0,5 - 4, et de préférence 0,8 - 2,
- acide mannuronique et/ou ses sels 0,5 - 4, et de préférence 0,8 - 2,
- hydroxyles saccharidiques estérifrés en acétate 0 - 8, et de préférence 0 - 6.

5. Hétéropolysaccharide (HP) selon l'une quelconque des revendications 1 à 4, **caractérisé en ce que** l'acide mannuronique se présente sous forme de sels.

6. Hétéropolysaccharide (HP) selon l'une quelconque des revendications 1 à 5, **caractérisé en ce qu'**il présente une masse molaire moyenne en poids (Mw) comprise entré 1.10⁵ et 8.10⁶ g/mol, de préférence comprise environ entre 8.10⁵ et 5.10⁶ g/mol.

7. Hétéropolysaccharide (HP) selon l'une quelconque des revendications 1 à 6, **caractérisé en ce qu'**il présente une masse molaire moyenne en poids (Mw) comprise environ entre 2,5.10⁶ et 4.10⁶ g/mol, les bornes étant inclues.

8. Hétéropolysaccharide (HP) selon l'une quelconque des revendications 1 à 7, **caractérisé en ce que** des solutions à 0,5 % en poids/poids dudit hétéropolysaccharide (HP) dans l'eau distillée à 23 °C, et à une fréquence de 1 Hz, conduisent à des valeurs de module élastique G' comprises entre 0,1 et 200 Pa, et celles de module de perte G" comprises entre 0,1 et 20 Pa.

9. Hétéropolysaccharide (HP) selon rune quelconque des revendications précédentes, **caractérisé en ce que** des solutions à 0,5 % en poids/poids dudit hétéropolysaccharide (HP) dans l'eau distillée à 23 °C, et à une fréquence de 1 Hz, conduit à des valeurs de G' comprises entre 20 et 200 Pa, et celles de G" comprises entre 0,5 et 15 Pa.

10. Hétéropolysaccharide (HP) selon l'une quelconque des revendications 1 à 9, **caractérisé en ce que** des solutions à 1 % en poids/poids dudit HP dans l'eau distillée contenant 0,5 % poids/poids de NaCl, à 23°C, conduit à des valeurs de viscosité en écoulement à un gradient de cisaillement de 0,1 s⁻¹ comprises entre 100 et 5000 Pa.s, et plus particuliérement entre 200 et 2000 Pa.s.

11. Hétéropolysaccharide (HP) selon l'une quelconque des revendications 1 à 10, **caractérisé en ce que** des solutions à 1 % en poids/poids dudit HP dans l'eau distillée contenant 0,5 % poids/poids de NaCI, à 23°C, conduit à des valeurs de viscosité en écoulement à un gradient de cisaillement de 10 s⁻¹ comprises entre 0,5 et 300 Pa.s, et plus particulièrement entre 5 et 150 Pa.s.

12. Procédé de préparation de l'hétéropolysaccharide (HP) tel que défini à l'une quelconque des revendications 1 à 11, **caractérisé en ce que** l'hétéropolysaccharide (HP) est séparé du moût de fermentation selon les étapes suivantes :
*i* - on soumet le moût de fin de fermentation à un traitement thermique entre 80°C et 120
°C pendant environ 10 à 60 minutes.
*ii* - on précipite ledit hétéropolysaccharide (HP) au moyen d'un liquide organique au moins partiellement miscible avec l'eau,
*iii* - on sépare l'hétéropolysaccharide (HP) du liquide organique.

13. Procédé selon la revendication 12, **caractérisé en ce que** dans l'étape (i) le moût soumis au traitement thermique présente un pH compris entre 6 et 8.

14. Procédé selon l'une des revendications 12 ou 13, **caractérisé en ce que** le moût issu de l'étape (i) est maintenu à la même température que la température du traitement thermique.

15. Procédé selon l'une quelconque des revendications 12 à 14, **caractérisé en ce que** dans l'étape *(ii)* la précipitation de l'hétéropolysaccharide (HP) par un liquide organique est réalisée en présence de sels, tels que les sulfates, les chlorures, ou les phosphates de sodium, de potassium, ou de calcium.

16. Procédé selon l'une quelconque des revendications 12 à 15, **caractérisé en ce que** dans l'étape *(ii)* la précipitation a lieu à une température comprise entre 40 et 60°C.

17. Souche de *Pseudomonas sp* déposée auprès de la CNCM, sous le numéro I - 2054 et également auprès de DSMZ, sous le numéro DSM 12295.

18. Culture pure de *Pseudomonas sp I - 2054* (ou *DSM 12295*).

19. Utilisation de l'hétéropolysaccharide (HP) tel que décrit à l'une quelconque des revendications 1 à 11 en tant qu'agent épaississant et/ou gélifiant.

20. Utilisation de l'hétéropolysaccharide (HP) tel que décrit à l'une quelconque des revendications 1 à 11, comme agent épaississant et/ou gélifiant dans des formulations alimentaires en des quantités comprises entre 0,01 à 5 % en poids, et de préférence entre 0,05 à 2 % en poids d'hétéropolysaccharide (HP) par rapport au poids de la composition ou formulation.

21. Utilisation de l'hétéropolysaccharide (HP) tel que décrit à l'une quelconque des revendications 1 à 11, pour l'obtention de gels sans ajout de cations supplémentaires au milieu.

22. Composition ou formulation comprenant l'hétéropolysaccharide (HP) tel que décrit à l'une quelconque des revendications 1 à 11.

## Patentansprüche

1. Heteropolysaccharid (HP), **dadurch gekennzeichnet, dass** es durch Fermentation eines Mediums erhalten werden kann, welches wenigstens einen Stamm *Pseudomonas sp I-2054* (oder *DSM 12295*), eine der Rekombinanten davon oder der Mutanten davon und eine Kohlenstoffquelle, die durch den Stamm, einer der Rekombinanten davon oder einer der Mutanten davon assimiliert werden kann, umfasst.

2. Heteropolysaccharid (HP) gemäß Anspruch 1, **dadurch gekennzeichnet, dass** es Motive von Glukose, Galactose und/oder deren Derivate, Mannuronsäure und/oder deren Salze umfasst, und wobei gewisse saccharidische Hydroxylgruppen davon in Form von Acetaten verestert sein können.

3. Heteropolysaccharid (HP) gemäß dem vorhergehenden Anspruch, **dadurch gekennzeichnet, dass** es die Motive in den folgenden Molverhältnissen umfasst, wobei als Referenz Galactose gleich 1 verwendet wird:
- Glukose und/oder dessen Derivate 0,2-5,
- Mannuronsäure und/oder dessen Salze 0,2-5,
- saccharidische Hydroxylgruppen, die mit Acetat verestert sind, 0-10.

4. Heteropolysaccharid (HP) gemäß einem der vorhergehenden Ansprüche, **dadurch gekennzeichnet, dass** es die Motive in den folgenden Molverhältnissen umfasst, wobei als Referenz Galactose gleich 1 verwendet wird:
- Glukose und/oder dessen Derivate 0,5-4, und bevorzugt 0,8-2,
- Mannuronsäure und/oder dessen Salze 0,5-4, und bevorzugt 0,8-2,
- saccharidische Hydroxylgruppen, die mit Acetat verestert sind, 0-8, und bevorzugt 0-6.

5. Heteropolysaccharid (HP) gemäß einem der Ansprüche 1-4, **dadurch gekennzeichnet, dass** die Mannuronsäure in Form von Salzen vorliegt.

6. Heteropolysaccharid (HP) gemäß einem der Ansprüche 1-5, **dadurch gekennzeichnet, dass** es eine Gewichtsmittel-Molekülmasse (Mw) aufweist, die zwischen 1·10⁵ und 8·10⁶ g/Mol liegt, bevorzugt zwischen ungefähr 8·10⁵ und 5·10⁶ g/Mol.

7. Heteropolysaccharid (HP) gemäß einem der Ansprüche 1-6, **dadurch gekennzeichnet, dass** es eine Gewichtsmittel-Molekülmasse (Mw) aufweist, die ungefähr zwischen 2,5·10⁶ und 4·10⁶ g/Mol liegt, einschließlich der Grenzen.

8. Heteropolysaccharid (HP) gemäß einem der Ansprüche 1-7, **dadurch gekennzeichnet, dass** 0,5 %-ige Lösungen in Gewicht/Gewicht des Heteropolysaccharids (HP) in destilliertem Wasser bei 23°C, und bei einer Frequenz von 1 Hz, zu Werten des Elastizitätsmoduls G', die zwischen 0,1 und 200 Pa liegen, und des Verlustmoduls G", die zwischen 0,1 und 20 Pa liegen, führen.

9. Heteropolysaccharid (HP) gemäß einem der vorhergehenden Ansprüche, **dadurch gekennzeichnet, dass** 0,5 %-ige Lösungen in Gewicht/Gewicht des Heteropolysaccharids (HP) in destilliertem Wasser bei 23°C, und bei einer Frequenz von 1 Hz, zu G'-Werten, die zwischen 20 und 200 Pa liegen, und zu G"-Werten, die zwischen 0,5 und 15 Pa liegen, führen.

10. Heteropolysaccharid (HP) gemäß einem der Ansprüche 1-9, **dadurch gekennzeichnet, dass** 1 %-ige Lösungen in Gewicht/Gewicht des HP in destilliertem Wasser, das 0,5 % Gewicht/Gewicht NaCl enthält, bei 23°C, zu Werten der Fließviskosität bei einem Schergradienten von 0,1 s⁻¹ führen, die zwischen 100 und 5000 Pa.s liegen, und insbesondere zwischen 200 und 2000 Pa.s.

11. Heteropolysaccharid (HP) gemäß einem der Ansprüche 1-10, **dadurch gekennzeichnet, dass** 1 %-ige Lösungen in Gewicht/Gewicht des HP in destilliertem Wasser, das 0,5 % Gewicht/Gewicht NaCl enthält, bei 23°C, zu Werten der Fließviskosität bei einem Schergradienten von 10 s⁻¹ führen, die zwischen 0,1 und 300 Pa.s liegen, und insbesondere zwischen 5 und 150 Pa.s.

12. Verfahren zur Herstellung des Heteropolysaccharids (HP) wie in einem der Ansprüche 1-11 definiert, **dadurch gekennzeichnet, dass** das Heteropolysaccharid (HP) vom Fermentationsmost gemäß den folgenden Schritten getrennt wird:
*i*- man unterwirft den Endfermentationsmost einer thermischen Behandlung zwischen 80°C und 120°C während ungefähr 10-60 Minuten,
*ii -* man fällt das Heteropolysaccharid (HP) mittels einer organischen Flüssigkeit, die wenigstens teilweise mit Wasser mischbar ist,
*iii-* man trennt das Heteropolysaccharid (HP) von der organischen Flüssigkeit.

13. Verfahren gemäß Anspruch 12, **dadurch gekennzeichnet, dass** in Schritt (*i*) der einer thermischen Behandlung unterworfene Most einen pH aufweist, der zwischen 6 und 8 liegt.

14. Verfahren gemäß einem der Ansprüche 12 oder 13, **dadurch gekennzeichnet, dass** der Most, der aus dem Schritt (*i*) stammt, bei der gleichen Temperatur wie die Temperatur der thermischen Behandlung gehalten wird.

15. Verfahren gemäß einem der Ansprüche 12-14, **dadurch gekennzeichnet, dass** in Schritt (*ii*) die Fällung des Heteropolysaccharids (HP) durch eine organische Flüssigkeit in Gegenwart von Salzen, wie Sulfaten, Chlorides oder Phosphaten von Natrium, Kalium oder Calcium durchgeführt wird.

16. Verfahren gemäß einem der Ansprüche 12-15, **dadurch gekennzeichnet, dass** in Schritt (*ii*) die Fällung bei einer Temperatur stattfindet, die zwischen 40 und 60°C liegt.

17. *Pseudomonas sp*-Stamm, hinterlegt bei CNCM, unter der Nr. I-2054 und gleichfalls bei DSMZ, unter der Nr. DSM 12295.

18. Reinkultur von *Pseudomonas Sp I*-*2054* (oder *DSM 12295*).

19. Verwendung des Heteropolysaccharids (HP) wie vorstehend in einem der Ansprüche 1-11 beschrieben als Verdickungsmittel und/oder Geliermittel.

20. Verwendung des Heteropolysaccharids (HP) wie in einem der Ansprüche 1-11 beschrieben als Verdickungsmittel und/oder Geliermittel in Nahrungsmittelformulierungen in Mengen zwischen 0,01-5 Gew.-%, und bevorzugt zwischen 0,05-2 Gew.-% Heteropolysaccharid (HP), bezogen auf das Gewicht der Zusammensetzung oder Formulierung.

21. Verwendung des Heteropolysaccharids (HP) wie in einem der Ansprüche 1-11 beschrieben zum Erhalten von Gelen ohne Zugabe von zusätzlichen Kationen zum Medium.

22. Zusammensetzung oder Formulierung, die ein Heteropolysaccharid (HP), wie in einem der Ansprüche 1-11 beschrieben, umfasst.

## Claims

1. A heteropolysaccharide (HP), **characterised in that** it can be obtained by the fermentation of a medium comprising at least one strain of *Pseudomonas sp I - 2054* (or *DSM 12295)*, one of the recombinants thereof, or the mutants thereof, and a source of carbon which can be assimilated by said strain, one of the recombinants thereof, or one of the mutants thereof.

2. A heteropolysaccharide (HP) according to Claim 1, **characterised in that** it comprises units of glucose, galactose; and/or derivatives thereof, mannuronic acid and/or salts thereof, and some saccharidic hydroxyls which can be esterified in the form of acetate.

3. A heteropolysaccharide (HP) according to the preceding claim, **characterised in that** it comprises said units in the following molar proportions, taking as reference galactose equal to 1:
- glucose and/or derivatives thereof 0.2 - 5,
- mannuronic acid and/or salts thereof 0.2 - 5,
- saccharidic hydroxyls esterified into acetate 0 - 10.

4. A heteropolysaccharide (HP) according to any one of the preceding claims, **characterised in that** it comprises said units in the following molar proportions, taking as reference galactose equal to 1:
- glucose and/or derivatives thereof 0.5 - 4, and preferably 0.8 - 2,
- mannuronic acid and/or salts thereof 0.5 - 4, and preferably 0.8 - 2,
- saccharidic hydroxyls esterified into acetate 0 - 8, and preferably 0 - 6.

5. A heteropolysaccharide (HP) according to any one of Claims 1 to 4, **characterised in that** the mannuronic acid is present in the form of salts.

6. A heteropolysaccharide (HP) according to any one of Claims 1 to 5, **characterised in that** it has an average molar mass by weight (Mw) of between 1.10⁵ and 8.10⁶ g/mol, preferably of between about 8.10⁵ and 5.10⁶ g/mol.

7. A heteropolysaccharide (HP) according to any one of Claims 1 to 6, **characterised in that** it has an average molar mass by weight (Mw) of between about 2.5.10⁶ and 4.10⁶ g/mol, inclusive of the limit values.

8. A heteropolysaccharide (HP) according to any one of Claims 1 to 7, **characterised in that** said solutions at 0.5% by weight/weight of said heteropolysaccharide (HP) in water distilled at 23°C and at a frequency of 1 Hz, result in elastic modulus values G' of between 0.1 and 200 Pa, and loss modulus values G" of between 0.1 and 20 Pa.

9. A heteropolysaccharide (HP) according to any one of the preceding claims, **characterised in that** said solutions at 0.5% by weight/weight of said heteropolysaccharide (HP) in water distilled at 23°C and at a frequency of 1 Hz, result in G' values of between 20 and 200 Pa and in G" values of between 0.5 and 15 Pa.

10. A heteropolysaccharide (HP) according to any one of Claims 1 to 9, **characterised in that** said solutions at 1% by weight/weight of said HP in distilled water containing 0.5% weight/weight of NaCl at 23°C, result in viscosity values of flow with a shear gradient of 0.1 s⁻¹ of between 100 and 5000 Pa.s, and, more particularly, of between 200 and 2000 Pa.s.

11. A heteropolysaccharide (HP) according to any one of Claims 1 to 10, **characterised in that** said solutions at 1% by weight/weight of said HP in distilled water containing 0.5% by weight/weight of NaCl at 23°C, result in viscosy values of flow with a shear gradient of 10 s⁻¹ of between 0.5 and 300 Pa.s, and, more particularly, of between 5 and 150 Pa.s.

12. A method for the production of the heteropolysaccharide (HP) as defined in any one of Claims 1 to 11, **characterised in that** the heteropolysaccharide (HP) is separated from the fermentation broth in the following steps :
*i* - the final fermentation broth is subjected to a heat treatment between 80°C and 120°C for about 10 to 60 minutes.
*ii* - said heteropolysaccharide (HP) is precipitated by means of an organic liquid which is at least partly miscible with water,
*iii* - the heteropolysaccharide (HP) is separated from the organic liquid.

13. A method according to Claim 12, **characterised in that** in step *(i)* the broth subjected to the heat treatment has a pH of between 6 and 8.

14. A method according to one of Claims 12 or 13, **characterised in that** the broth resulting from step (*i*) is kept at the same temperature as the temperature of the heat treatment.

15. A method according to any one of Claims 12 to 14, **characterised in that** in step (*ii*) precipitation of the heteropolysaccharide (HP) by an organic liquid is carried out in the presence of salts, such as sulphates, chlorides, or sodium, potassium or calcium phosphates.

16. A method according to any one of Claims 12 to 15, **characterised in that** in step (*ii*) precipitation takes place at a temperature of between 40 and 60°C.

17. A *Pseudomonas sp* strain, registered at the CNCM under No. 1 - 2054 and also at the DSMZ under No. DSM 12295.

18. A pure culture of *Pseudomonas sp I - 2054* (or DSM 12295).

19. Use of the heteropolysaccharide (HP) as described in any one of Claims 1 to 11 as a thickening agent and/or gelling agent.

20. Use of the heteropolysaccharide (HP) as described in any one of Claims 1 to 11, as a thickening agent and/or gelling agent in alimentary formulations with quantities of between 0.01 and 5% by weight, and preferably of between 0.05 and 2% by weight of heteropolysaccharide (HP) in relation to the weight of the composition or formulation.

21. Use of the heteropolysaccharide (HP) such as described in any one of Claims I to 11 for obtaining gels without adding extra cations to the medium.

22. A composition or formulation comprising the heteropolysaccharide (HP) such as described in any one of Claims 1 to 11.
